# EUROPEAN PATENT SPECIFICATION

(11) **EP 1 370 220 B1**
(45) Date of publication and mention of the grant of the patent: **06.06.2007**
(21) Application number: 02730000.3
(22) Date of filing: 13.03.2002
(51) Int. Cl.: A61Q 5/02, A61K 8/04, A61K 8/362, A61K 8/73, A61K 8/46, A61K 8/44

(54) **SHAMPOO COMPOSITIONS**
HAARWASCHMITTEL
COMPOSITIONS DE SHAMPOOING

(30) Priority: 21.03.2001 EP 01302641
(43) Date of publication of application: 17.12.2003
(73) Proprietor: UNILEVER PLC, London EC4P 4BQ (GB); UNILEVER N.V., 3013 AL Rotterdam (NL)
(72) Inventor: FAIRLEY, Peter, Lever Faberge France, 60881 Le Meux (FR); SHAW, Neil Scott, Unilever Research Port Sunlight, Wirral, Merseyside CH63 3JW (GB); STEER, David Charles, Heswall, Wirral, Merseyside CH60 6RS (GB)
(74) Representative: James, Helen Sarah
(86) International application number: PCT/EP2002/002743
(87) International publication number: WO 2002/089753

(56) References cited:
- EP-A- 0 468 721
- EP-A- 0 562 638
- WO-A-93/08787
- WO-A-97/35549
- WO-A-98/18443
- WO-A-99/32070
- US-A- 5 344 643
- US-A- 5 876 705

## Description

### FIELD OF THE INVENTION

This invention relates to shampoo compositions containing hair conditioning agents, more particularly oily conditioning agents.

### BACKGROUND AND PRIOR ART

Shampoo compositions which provide a combination of cleansing and conditioning to the hair are well know in the art. Such shampoo compositions typically comprise one or more surfactants for shampooing or cleansing purposes and one or more conditioning agents. The purpose of the conditioning agent is to make the hair easier to comb when wet and more manageable when dry, e.g. less static and fly-away. Typically, these conditioning agents are either water-insoluble oily materials or cationic materials, i.e. polymers or surfactants.

Amongst the most popular conditioning agents used in shampoo products are silicone polymers, present in the shampoo as emulsion droplets. Conditioning is achieved by the silicone being deposited onto the hair resulting in the formation of a film. Whilst the silicone film gives excellent conditioning, for example wet comb properties, the repeated use of these compositions can lead to a build up of silicone and undesirable affects such as a heavy, oily feel to the hair.

A problem associated with use of cationic polymers and cationic surfactants as conditioning agents is that the anionic surfactants commonly employed in shampoo compositions can interact with the cationic conditioning agents resulting in poorer performance of the conditioning agent and/or the cleansing surfactant. Companies have attempted to overcome this problem by employing nonionic, amphoteric and/or cationic co-surfactants as cleansing agents, but these systems do not provide as high a level of cleansing or foaming as do anionic surfactant systems.

Another class of conditioning agents used in shampoo compositions is non-silicone water-insoluble oily materials. Such oily materials have been used to a far lesser degree than silicone and cationic conditioning agents. Two of the main reasons for this are the incompatibility of such materials with shampoo surfactants, especially anionic surfactants, and the inherent instability of emulsions of these oily materials in aqueous-based shampoo compositions. This often results in a lack of homogeneity in the shampoo composition and/or inadequate shelf-life.

Accordingly, there is a need for shampoo compositions containing non-silicone oily conditioning agents and which deliver both good cleansing and conditioning performance, and which are also stable. We have now found that when employing an oily conditioning agent as an emulsion in an aqueous shampoo composition, both good stability and deposition of the oily conditioning agent can be achieved by controlling the particle size of the conditioning agent.

EP 074,264 (Unilever) discloses aqueous conditioning shampoos having improved foaming quality comprising (i) an anionic or amphoteric detergent, (ii) a cationic or nonionic conditioning agent, and (iii) a water-miscible saccharide. Suitable conditioning agents are said to include silicones, resinous materials, waxy materials and oily materials. A shampoo composition comprising light mineral oil is disclosed in Example XI.

WO 93/08787 (Procter & Gamble) discloses an aqueous conditioning shampoo comprising (i) an anionic surfactant, (ii) a dispersed, insoluble, non-volatile, nonionic silicone conditioning agent, (iii) a water-soluble cationic organic hair conditioning polymer of specified charge density, and (iv) an organic, non-volatile, water-insoluble, liquid selected from the group consisting of hydrocarbon oils, fatty ester and mixtures thereof.

WO 97/35549 (Procter & Gamble) discloses an aqueous conditioning shampoo comprising (i) a specific surfactant component comprising an ethoxyated alkyl sulphate surfactant having from about 1 to 8 moles of ethoxylation and an amphoteric surfactant, (ii) a cationic cellulosic polymer of defined molecular weight and charge density, (iii) a water-insoluble non-volatile silicone conditioning agent having an average particle size below 4 µm, and (iv) an organic, non-volatile, water-insoluble liquid solubilised in the surfactant component and having a defined viscosity and being selected from hydrocarbon oils and fatty esters having boiling point at ambient pressures of 250°C or higher, or mixtures thereof.

US 5,344,643 (Dowbrands) discloses an aqueous shampoo composition containing (i) an anionic surfactant, (ii) an oily, water-insoluble conditioning agent, (iii) a carboxyvinyl polymer suspending and stabilising agent for the oily conditioning agent, and (iv) a cationic conditioning agent. There is no disclosure of the size of emulsion droplets of the oily conditioning agents other than the statement that how the oily conditioning agent is mixed into shampoo composition is not critical and may be effected by either simultaneous mixing or by conventional batch mixing (col. 12, lines 52-55). In other words, US 5,344,643 teaches that the size of the oil droplets is not important.

None of the above documents disclose the droplet size of a non-silicone oily conditioning agent in the emulsion. Furthermore, none of the above documents mention that the droplet size might be relevant or important to stability and deposition. Indeed, US 5,344,643 teaches to the contrary.

The present inventors have now found that when the non-silicone oily conditioning agent has a droplet in a specific range, namely from 0.4 to 4 µm, the shampoo conditioning compositions have both good stability and deposition performance.

EP 093,601 (Unilever) discloses washing compositions, including shampoo compositions, which deposit water-insoluble particles on the surface being washed, the compositions comprising (i) an anionic surfactant, (ii) the particulate substance to be deposited, and (iii) a water-soluble cationic non-cellulosic polymer which enhances the deposition of the particulate substance, the cationic polymer being more specifically defined in terms of charge density and function. A wide variety of water-insoluble particles can be employed. These include substances having an average particle diameter of from about 0.2 to about 50 µm. Shampoo compositions may include particles of water-insoluble oil as conditioning agent, such oil being emulsified in the composition.

EP 529,883 (Unilever) discloses cosmetic compositions having good mechanical stability, high optical transparency or translucency, and excellent conditioning ability comprising a microemulsified conditioning oil of particle size no more than 0.15 µm, preferably no more than 0.1 µm, in combination with a deposition polymer. The only conditioning oils specifically mentioned are silicone oils.

EP 562,638 (Unilever) discloses an aqueous conditioning shampoo comprising (i) an anionic surfactant, (ii) a water-soluble cationic conditioning compound, (iii) a suspended water-insoluble conditioning compound of defined viscosity, specific gravity and refractive index, and (iv) a suspending agent for the water-insoluble conditioning agent. Suitable conditioning agents include emollient esters, hydrocarbons, such as low viscosity mineral oils. According to the teaching of this document, in order to get good deposition and stability, the water-insoluble conditioning compound should have a particle size in the range from at least 5, preferably at least 10, to 100 µm. If the particle size is less that about 5 µm, according to this document, the total surface area is too great and the conditioning compound is rinsed away with the anionic surfactants.

WO 98/22087 (L'Oreal) discloses shampoo conditioning compositions comprising oil nanoemulsion consisting of an oil-in water emulsion having oil droplets of mean size less than 150 nm. The oil comprises an amphiphilic lipid phase including at least one nonionic amphiphilic lipid that is liquid at an ambient temperature of less than 45°C.

The present inventors have now found that aqueous shampoo compositions containing an oily conditioning agent having a droplet size in the range from 0.4 to 4 µm exhibit both good stability and deposition of the conditioning agent. Furthermore, when the droplet size is outside of this range, either the stability or the deposition is compromised to an unacceptable degree.

### SUMMARY OF THE INVENTION

This invention provides an aqueous shampoo composition comprising, in addition to water:
1) a cleansing surfactant;
   ii) a dispersed non-volatile, non-silicone, water-insoluble, oily conditioning agent comprising a triglyceride, the average D_{3,2} droplet size of which is in the range from 0.8 to 4 µm; and
   iii) a cationic polymer.

### DETAILED DESCRIPTION AND PREFERRED EMBODIMENTS

Unless specified otherwise, all wt% values quoted hereinafter are percentages by weight based on total weight of the shampoo composition.

### (i) Oily Conditioning Agent

Compositions according to the present invention comprise a dispersed, non-volatile, water-insoluble oily conditioning agent.

This component will be dispersed in the composition in the form of droplets, which form a separate, discontinuous phase from the aqueous, continuous phase of the composition. In other words, the oily conditioning agent will be present in the shampoo composition form of an oil-in-water emulsion.

When components are present in the compositions of the present invention in droplet form, the droplets may be liquid, semi-solid or solid in nature, so long as they are substantially uniformly dispersed in the fully formulated product. The droplets of oily conditioning agent are preferably present as either liquid or semi-solid droplets, more preferably as liquid droplets.

As used herein, the term "conditioning agent" includes any material, which is used to give a particular conditioning benefit to hair. For example, suitable materials are those which deliver one or more benefits relating to shine, softness, combability, wet-handling, anti-static properties, protection against damage, body, volume, stylability and manageability.

By "insoluble" is meant that the material is not soluble in water (distilled or equivalent) at a concentration of 0.1% (w/w), at 25°C.

The D_{3,2} average droplet size of the oily conditioning component is at least 0.8, and preferably at least 1 µm. Additionally, the D_{3,2} average droplet size of the oily conditioning component is no greater than 4. µm, preferably no greater than 3.5 µm, more preferably no greater than 3 µm. It should be noted that suitable droplet size ranges include any maximum D_{3,2} average droplet size associated with any minimum D_{3,2} average droplet size. Preferred ranges are from 0.8 to 3.5 µm, yet more preferably from 0.8 to 3 µm, and most preferably from 1 to 3.5 µm, for example from 1 to 3 µm.

When using certain oily conditioning agents, especially, for example, lower molecular weight agents, the lower limit of droplet size may be higher, for example at least 0.8, more preferably at least 1 µm. Increasing the lower limit gives better stability of such compositions.

The D_{3,2} average droplet size may be measured by means of a laser light scattering technique, using a-2600D Particle Sizer from Malvern Instruments.

The present inventors have found that aqueous shampoo compositions containing a non-silicone oily conditioning agent having a droplet size in the range from 0.8 to 4 µm exhibit both good stability and deposition of the conditioning agent. Preferably, the compositions of the present invention have a deposition efficiency with respect to the oily conditioning agent of at least 8%, more preferably at least 9%, yet more preferably at least 10%. The deposition efficiency is the proportion of the oily conditioning agent in the shampoo which is actually deposited in the hair (w/w).

One measurement of stability is the maintenance of viscosity over time. Preferably, the compositions of the present invention lose less than 20%, more preferably less than 10%, and most preferably less than 5% of their viscosity on storage at 37°C over a period of 4 weeks. The viscosity of the shampoo composition is measured at 25°C with a Brookfield Viscometer (e.g. Brookfield RV) using spindle 4 operating at 20 rpm.

The oily conditioning agent may suitably be selected from oily or fatty materials, and mixtures thereof.

Oily or fatty materials are preferred conditioning agents in the shampoo compositions of the invention for adding shine to the hair and also enhancing dry combing and dry hair feel.

Preferred oily and fatty materials will generally have a viscosity of less than 5 Pa.s, more preferably less than 1 Pa.s, and most preferably less than 0.5 Pa.s, e.g. 0.1 Pa.s and under as measured at 25°C with a Brookfield Viscometer (e.g. Brookfield RV) using spindle 3 operating at 100 rpm.

Oily and fatty materials with higher viscosities may be used. For example, materials with viscosities as high as 65 Pa.s may be used. The viscosity of such materials (i.e. materials with viscosities of 5 Pa.s and greater) can be measured by means of a glass capillary viscometer as set out further in Dow Corning Corporate Test Method CTM004, July 20 1970.

Suitable oily or fatty materials are selected from hydrocarbon oils, fatty esters and mixtures thereof.

Hydrocarbon oils include cyclic hydrocarbons, straight chain aliphatic hydrocarbons (saturated or unsaturated), and branched chain aliphatic hydrocarbons (saturated or unsaturated). Straight chain hydrocarbon oils will preferably contain from about 12 to about 30 carbon atoms. Branched chain hydrocarbon oils can and typically may contain higher numbers of carbon atoms. Also suitable are polymeric hydrocarbons of alkenyl monomers, such as C₂-C₆ alkenyl monomers. These polymers can be straight or branched chain polymers. The straight chain polymers will typically be relatively short in length, having a total number of carbon atoms as described above for straight chain hydrocarbons in general. The branched chain polymers can have substantially higher chain length. The number average molecular weight of such materials can vary widely, but will typically be up to about 2000, preferably from about 200 to about 1000, more preferably from about 300 to about 600.

Specific examples of suitable hydrocarbon oils include paraffin oil, mineral oil, saturated and unsaturated dodecane, saturated and unsaturated tridecane, saturated and unsaturated tetradecane, saturated and unsaturated pentadecane, saturated and unsaturated hexadecane, and mixtures thereof. Branched-chain isomers of these compounds, as well as of higher chain length hydrocarbons, can also be used. Exemplary branched-chain isomers are highly branched saturated or unsaturated alkanes, such as the permethyl-substituted isomers, e.g., the permethyl-substituted isomers of hexadecane and eicosane, such as 2, 2, 4, 4, 6, 6, 8, 8-dimethyl-10-methylundecane and 2, 2, 4, 4, 6, 6-dimethyl-8-methylnonane, sold by Permethyl Corporation. A further example of a hydrocarbon polymer is polybutene, such as the copolymer of isobutylene and butene. A commercially available material of this type is L-14 polybutene from Amoco Chemical Co. (Chicago, Ill., U.S.A.).

Particularly preferred hydrocarbon oils are the various grades of mineral oils. Mineral oils are clear oily liquids obtained from petroleum oil, from which waxes have been removed, and the more volatile fractions removed by distillation. The fraction distilling between 250°C to 300°C is termed mineral oil, and it consists of a mixture of hydrocarbons ranging from C₁₆H₃₄ to C₂₁H₄₄. Suitable commercially available materials of this type include Sirius M85 and Sirius M125, all available from Silkolene.

Suitable fatty esters are characterised by having at least 10 carbon atoms, and include esters with hydrocarbyl chains derived from fatty acids or alcohols, e.g., monocarboxylic acid esters, polyhydric alcohol esters, and di- and tricarboxylic acid esters. The hydrocarbyl radicals of the fatty esters hereof can also include or have covalently bonded thereto other compatible functionalities, such as amides and alkoxy moieties, such as ethoxy or ether linkages.

Monocarboxylic acid esters include esters of alcohols and/or acids of the formula R'COOR in which R' and R independently denote alkyl or alkenyl radicals and the sum of carbon atoms in R' and R is at least 10, preferably at least 20.

Specific examples include, for example, alkyl and alkenyl esters of fatty acids having aliphatic chains with from about 10 to about 22 carbon atoms, and alkyl and/or alkenyl fatty alcohol carboxylic acid esters having an alkyl and/or alkenyl alcohol-derived aliphatic chain with about 10 to about 22 carbon atoms, benzoate esters of fatty alcohols having from about 12 to 20 carbon atoms.

The monocarboxylic acid ester need not necessarily contain at least one chain with at least 10 carbon atoms, so long as the total number of aliphatic chain carbon atoms is at least 10. Examples include isopropyl isostearate, hexyl laurate, isohexyl laurate, isohexyl palmitate, isopropyl palmitate, decyl oleate, isodecyl oleate, hexadecyl stearate, decyl stearate, isopropyl isostearate, dihexyldecyl adipate, lauryl lactate, myristyl lactate, cetyl lactate, oleyl stearate, oleyl oleate, oleyl myristate, lauryl acetate, cetyl propionate, and oleyl adipate.

Di- and trialkyl and alkenyl esters of carboxylic acids can also be used. These include, for example, esters of C₄-C₈ dicarboxylic acids such as C₁-C₂₂ esters (preferably C₁-C₆) of succinic acid, glutaric acid, adipic acid, hexanoic acid, heptanoic acid, and octanoic acid. Examples include diisopropyl adipate, diisohexyl adipate, and diisopropyl sebacate. Other specific examples include isocetyl stearoyl stearate, and tristearyl citrate.

Polyhydric alcohol esters include alkylene glycol esters, for example ethylene glycol mono and di-fatty acid esters, diethylene glycol mono- and di-fatty acid esters, polyethylene glycol mono- and di-fatty acid esters, propylene glycol mono- and di-fatty acid esters, polypropylene glycol monooleate, polypropylene glycol monostearate, ethoxylated propylene glycol monostearate, polyglycerol poly-fatty acid esters, ethoxylated glyceryl monostearate, 1,3-butylene glycol monostearate, 1,3-butylene glycol distearate, polyoxyethylene polyol fatty acid ester, sorbitan fatty acid esters, polyoxyethylene sorbitan fatty acid esters and mono-, di-and triglycerides.

Particularly preferred fatty esters are mono-, di- and triglycerides, more specifically the mono-, di-, and triesters of glycerol and long chain carboxylic acids such as C₁-C₂₂ carboxylic acids. A variety of these types of materials can be obtained from vegetable and animal fats and oils, such as coconut oil, castor oil, safflower oil, sunflower oil, cottonseed oil, corn oil, olive oil, cod liver oil, almond oil, avocado oil, palm oil, sesame oil, peanut oil, lanolin and soybean oil. Synthetic oils include triolein and tristearin glyceryl dilaurate.

Specific examples of preferred materials include cocoa butter, palm stearin, sunflower oil, soyabean oil and coconut oil.

The oily or fatty material is typically present at a level of from 0.05 to 10, preferably from 0.2 to 5, more preferably from about 0.5 to 3 wt%.

### (ii) Surfactant

Compositions according to the present invention comprise at least one cleansing surfactant.

### Anionic Cleansing Surfactant

Shampoo compositions according to the invention will typically comprise one or more anionic cleansing surfactants which are cosmetically acceptable and suitable for topical application to the hair.

Examples of suitable anionic cleansing surfactants are the alkyl sulphates, alkyl ether sulphates, alkaryl sulphonates, alkanoyl isethionates, alkyl succinates, alkyl sulphosuccinates, N-alkyl sarcosinates, alkyl phosphates, alkyl ether phosphates, alkyl ether carboxylates, and alpha-olefin sulphonates, especially their sodium, magnesium, ammonium and mono-, di- and triethanolamine salts. The alkyl and acyl groups generally contain from 8 to 18 carbon atoms and may be unsaturated. The alkyl ether sulphates, alkyl ether phosphates and alkyl ether carboxylates may contain from 1 to 10 ethylene oxide or propylene oxide units per molecule.

Typical anionic cleansing surfactants for use in shampoo compositions of the invention include sodium oleyl succinate, ammonium lauryl sulphosuccinate, ammonium lauryl sulphate, sodium dodecylbenzene sulphonate, triethanolamine dodecylbenzene sulphonate, sodium cocoyl isethionate, sodium lauryl isethionate and sodium N-lauryl sarcosinate. The most preferred anionic surfactants are sodium lauryl sulphate, sodium lauryl ether sulphate(n)EO, (where n ranges from 1 to 3), ammonium lauryl sulphate and ammonium lauryl ether sulphate(n)EO, (where n ranges from 1 to 3).

Mixtures of any of the foregoing anionic cleansing surfactants may also be suitable.

The total amount of anionic cleansing surfactant in shampoo compositions of the invention is generally from 5 to 30, preferably from 6 to 20, more preferably from 8 to 16 wt%.

### Co-surfactant

The shampoo composition can optionally include co-surfactants, to help impart aesthetic, physical or cleansing properties to the composition.

A preferred example is an amphoteric or zwitterionic surfactant, which can be included in an amount ranging from 0 to about 8, preferably from 1 to 4 wt%.

Examples of amphoteric and zwitterionic surfactants include alkyl amine oxides, alkyl betaines, alkyl amidopropyl betaines, alkyl sulphobetaines (sultaines), alkyl glycinates, alkyl carboxyglycinates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in shampoos of the invention include lauryl amine oxide, cocodimethyl sulphopropyl betaine and preferably lauryl betaine, cocamidopropyl betaine and sodium cocamphopropionate.

Another preferred example is a nonionic surfactant, which can be included in an amount ranging from 0 to 8, preferably from 2 to 5 wt%.

For example, representative nonionic surfactants that can be included in shampoo compositions of the invention include condensation products of aliphatic (C₈ - C₁₈) primary or secondary linear or branched chain alcohols or phenols with alkylene oxides, usually ethylene oxide and generally having from 6 to 30 ethylene oxide groups.

Other representative nonionic surfactants include mono- or di-alkyl alkanolamides. Examples include coco mono- or diethanolamide and coco mono-isopropanolamide.

Further nonionic surfactants which can be included in shampoo compositions of the invention are the alkyl polyglycosides (APGs). Typically, the APG is one which comprises an alkyl group connected (optionally via a bridging group) to a block of one or more glycosyl groups. Preferred APGs are defined by the following formula:

RO-(G)ₙ

wherein R is a branched or straight chain alkyl group which may be saturated or unsaturated and G is a saccharide group.

R may represent a mean alkyl chain length of from about C₅ to about C₂₀. Preferably R represents a mean alkyl chain length of from about C₈ to about C₁₂. Most preferably the value of R lies between about 9.5 and about 10.5. G may be selected from C₅ or C₆ monosaccharide residues, and is preferably a glucoside. G may be selected from the group comprising glucose, xylose, lactose, fructose, mannose and derivatives thereof. Preferably G is glucose.

The degree of polymerisation, n, may have a value of from about 1 to about 10 or more. Preferably, the value of n lies in the range of from about 1.1 to about 2. Most preferably the value of n lies in the range of from about 1.3 to about 1.5.

Suitable alkyl polyglycosides for use in the invention are commercially available and include for example those materials identified as: Oramix NS10 ex Seppic; Plantaren 1200 and Plantaren 2000 ex Henkel.

Other sugar-derived nonionic surfactants which can be included in shampoo compositions of the invention include the C₁₀-C₁₈ N-alkyl (C₁-C₆) polyhydroxy fatty acid amides, such as the C₁₂-C₁₈ N-methyl glucamides, as described for example in WO 92 06154 and US 5 194 639, and the N-alkoxy polyhydroxy fatty acid amides, such as C₁₀-C₁₈ N-(3-methoxypropyl) glucamide.

The shampoo composition can also optionally include one or more cationic co-surfactants included in an amount ranging from 0.01 to 10, more preferably from 0.05 to 5, most preferably from 0.05 to 2 wt%.

Examples of suitable cationic surfactants include:
quaternary ammonium hydroxides, e.g., tetramethylammonium hydroxide, alkyltrimethylammonium hydroxides wherein the alkyl group has from about 8 to 22 carbon atoms, for example octyltrimethylammonium hydroxide, dodecyltrimethy- ammonium hydroxide, hexadecyltrimethylammonium hydroxide, cetyltrimethylammonium hydroxide, octyldimethylbenzylammonium hydroxide, decyldimethylbenzylammonium hydroxide, stearyldi-methylbenzylammonium hydroxide, didodecyldimethylammonium hydroxide, dioctadecyldimethylammonium hydroxide, tallow trimethylammonium hydroxide, cocotrimethylammonium hydroxide, and the corresponding salts thereof, e.g., chlorides Cetylpyridinium hydroxide or salts thereof, e.g., chloride Quaternium -5 Quaternium -31 Quaternium -18 and mixtures thereof.

The total amount of surfactant (including any co-surfactant, and/or any emulsifier, e.g. for any silicone component if present) in shampoo compositions of the invention is generally from 0.1 to 50, preferably from 5 to 30, more preferably from 10 to 25 wt%.

### (iii) Cationic Polymer

Compositions according to the present invention comprise a cationic polymer for enhancing conditioning performance of the shampoo.

The cationic polymer may be a homopolymer or be formed from two or more types of monomers. The molecular weight of the polymer will generally be between 5 000 and 10 000 000, typically at least 10 000 and preferably in the range 100 000 to about 2 000 000. The polymers will have cationic nitrogen containing groups such as quaternary ammonium or protonated amino groups, or a mixture thereof.

The cationic nitrogen-containing group will generally be present as a substituent on a fraction of the total monomer units of the cationic polymer. Thus when the polymer is not a homopolymer it can contain spacer non-cationic monomer units. Such polymers are described in the CTFA Cosmetic Ingredient Directory, 3rd edition. The ratio of the cationic to non-cationic monomer units is selected to give a polymer having a cationic charge density in the required range.

Suitable cationic conditioning polymers include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general secondary and tertiary amines, especially tertiary, are preferred.

Amine substituted vinyl monomers and amines can be polymerized in the amine form and then converted to ammonium by quaternization.

The cationic conditioning polymers can comprise mixtures of monomer units derived from amine- and/or quaternary ammonium-substituted monomer and/or compatible spacer monomers.

Suitable cationic conditioning polymers include, for example:
- copolymers of 1-vinyl-2-pyrrolidine and 1-vinyl-3-methyl-imidazolium salt (e.g. chloride salt), referred to in the industry by the Cosmetic, Toiletry, and Fragrance Association, (CTFA) as Polyquaternium-16. This material is commercially available from BASF Wyandotte Corp. (Parsippany, NJ, USA) under the LUVIQUAT tradename (e.g. LUVIQUAT FC 370);
- copolymers of 1-vinyl-2-pyrrolidine and dimethylaminoethyl methacrylate, referred to in the industry (CTFA) as Polyquaternium-11. This material is available commercially from Gaf Corporation (Wayne, NJ, USA) under the GAFQUAT tradename (e.g., GAFQUAT 755N);
- cationic diallyl quaternary ammonium-containing polymers including, for example, dimethyldiallyammonium chloride homopolymer and copolymers of acrylamide and dimethyldiallylammonium chloride, referred to in the industry (CTFA) as Polyquaternium 6 and Polyquaternium 7, respectively;
- mineral acid salts of amino-alkyl esters of homo-and copolymers of unsaturated carboxylic acids having from 3 to 5 carbon atoms, (as described in U.S. Patent 4,009,256);
- cationic polyacrylamides(as described in WO95/22311)

Other cationic conditioning polymers that can be used include cationic polysaccharide polymers, such as cationic cellulose derivatives, cationic starch derivatives, and cationic guar gum derivatives. Suitably, such cationic polysaccharide polymers have a charge density in the range from 0.1 to 4 meq/g.

Cationic polysaccharide polymers suitable for use in compositions of the invention include those of the formula:

A-O-[R-N⁺(R¹) (R²) (R³)X⁻],

wherein: A is an anhydroglucose residual group, such as a starch or cellulose anhydroglucose residual. R is an alkylene, oxyalkylene, polyoxyalkylene, or hydroxyalkylene group, or combination thereof. R¹, R² and R³ independently represent alkyl, aryl, alkylaryl, arylalkyl, alkoxyalkyl, or alkoxyaryl groups, each group containing up to about 18 carbon atoms. The total number of carbon atoms for each cationic moiety (i.e., the sum of carbon atoms in R¹, R² and R³) is preferably about 20 or less, and X is an anionic counterion.

Cationic cellulose is available from Amerchol Corp. (Edison, NJ, USA) in their Polymer JR (trade mark) and LR (trade mark) series of polymers, as salts of hydroxyethyl cellulose reacted with trimethyl ammonium substituted epoxide, referred to in the industry (CTFA) as Polyquaternium 10. Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from Amerchol Corp. (Edison, NJ, USA) under the tradename Polymer LM-200.

Other suitable cationic polysaccharide polymers include quaternary nitrogen-containing cellulose ethers (e.g. as described in U.S. Patent 3,962,418), and copolymers of etherified cellulose and starch (e.g. as described in U.S. Patent 3,958,581).

A particularly suitable type of cationic polysaccharide polymer that can be used is a cationic guar gum derivative, such as guar hydroxypropyltrimonium chloride (commercially available from Rhone-Poulenc in their JAGUAR trademark series).

Examples are JAGUAR C13S, which has a low degree of substitution of the cationic groups and high viscosity. JAGUAR C15, having a moderate degree of substitution and a low viscosity, JAGUAR C17 (high degree of substitution, high viscosity), JAGUAR C16, which is a hydroxypropylated cationic guar derivative containing a low level of substituent groups as well as cationic quaternary ammonium groups, and JAGUAR 162 which is a high transparency, medium viscosity guar having a low degree of substitution.

Preferably the cationic conditioning polymer is selected from cationic cellulose and cationic guar derivatives. Particularly preferred cationic polymers are JAGUAR C13S, JAGUAR C15, JAGUAR C17 and JAGUAR C16 and JAGUAR C162.

The cationic conditioning polymer will generally be present in compositions of the invention at levels of from 0.01 to 5, preferably from 0.05 to 1, more preferably from 0.08 to 0.5 wt%.

### OPTIONAL COMPONENTS

As optional components for inclusion in compositions according to the invention may be mentioned the following conventional adjunct materials known for use in shampoo compositions: suspending agents, thickeners, pearlescing agents, opacifiers, salts, perfumes, buffering agents, colouring agents, emollients, moisturisers, foam stabilisers, sunscreen materials, antimicrobial agents, preservatives, antioxidants.

### Suspending Agents

In a preferred embodiment, the composition according to the invention furthers comprises from 0.1 to 5 wt% of a suspending agent for the oily conditioning agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives. Polyacrylic acid is available commercially as Carbopol 420, Carbopol 488 or Carbopol 493. Polymers of acrylic acid cross-linked with a polyfunctional agent may also be used, they are available commercially as Carbopol 910, Carbopol 934, Carbopol 940, Carbopol 941 and Carbopol 980. An example of a suitable copolymer of a carboxylic acid containing a monomer and acrylic acid esters is Carbopol 1342. All Carbopol (trade mark) materials are available from Goodrich.

Suitable cross-linked polymers of acrylic acid and acrylate esters are Pemulen TR1 or Pemulen TR2. A suitable heteropolysaccharide gum is xanthan gum, for example that available as Kelzan mu.

The suspending agent for the oily conditioning agent is preferably a polymeric suspending agent.

### Silicone Conditioning Agent

The compositions of the invention can contain, in addition to the non-silicone oily conditioning agent, emulsified droplets of a silicone conditioning agent, for enhancing conditioning performance. The silicone is insoluble in the aqueous matrix of the composition and so is present in an emulsified form, with the silicone present as dispersed droplets.

Suitable silicones include polydiorganosiloxanes, in particular polydimethylsiloxanes which have the CTFA designation dimethicone. Also suitable for use compositions of the invention (particularly shampoos and conditioners) are polydimethyl siloxanes having hydroxyl end groups, which have the CTFA designation dimethiconol. Also suitable for use in compositions of the invention are silicone gums having a slight degree of cross-linking, as are described for example in WO 96/31188. These materials can impart body, volume and stylability to hair, as well as good wet and dry conditioning.

The viscosity of the emulsified silicone itself (not the emulsion or the final hair conditioning composition) is typically at least 10,000 cst. In general we have found that conditioning performance increases with increased viscosity. Accordingly, the viscosity of the silicone itself is preferably at least 60,000 cst, most preferably at least 500,000 cst, ideally at least 1,000,000 cst. Preferably the viscosity does not exceed 10⁹ cst for ease of formulation. All viscosities referred to here are at 25°C.

Emulsified silicones for use in the shampoo compositions of the invention will typically have an average silicone droplet size in the composition of less than 30, preferably less than 20, more preferably less than 10 µm. We have found that reducing the droplet size generally improves conditioning performance. Most preferably the average silicone droplet size of the emulsified silicone in the composition is less than 2 µm, ideally it ranges from 0.01 to 1 µm. Silicone emulsions having an average silicone droplet size of ≤ 0.15 µm are generally termed microemulsions.

Suitable silicone emulsions for use in the invention are also commercially available in a pre-emulsified form.

Examples of suitable pre-formed emulsions include emulsions DC2-1766, DC2-1784, and microemulsions DC2-1865 and DC2-1870, all available from Dow Corning. These are all emulsions/microemulsions of dimethiconol. Cross-linked silicone gums are also available in a pre-emulsified form, which is advantageous for ease of formulation. A preferred example is the material available from Dow Corning as DC X2-1787, which is an emulsion of cross-linked dimethiconol gum. A further preferred example is the material available from Dow Corning as DC X2-1391, which is a microemulsion of cross-linked dimethiconol gum.

A further preferred class of silicones for inclusion in shampoos and conditioners of the invention are amino functional silicones. By "amino functional silicone" is meant a silicone containing at least one primary, secondary or tertiary amine group, or a quaternary ammonium group.

Examples of suitable amino functional silicones include:
(i) polysiloxanes having the CTFA designation "amodimethicone", and the general formula:

   HO-[Si(CH₃)₂-O-]ₓ-[Si(OH)(CH₂CH₂CH₂-NH-CH₂CH₂NH₂)-O-]_{y}-H

   in which x and y are numbers depending on the molecular weight of the polymer, generally such that the molecular weight is between about 5,000 and 500,000.
(ii) polysiloxanes having the general formula:

   R'ₐG₃₋ₐ-Si(OSiG₂)ₙ-(OSiG_{b}R'_{2-b})ₘ-O-SiG₃₋ₐ-R'ₐ

   in which:
   G is selected from H, phenyl, OH or C₁₋₈ alkyl, e.g. methyl;
   a is 0 or an integer from 1 to 3, preferably 0;
   b is 0 or 1, preferably 1;
   m and n are numbers such that (m + n) can range from 1 to 2000, preferably from 50 to 150;
   m is a number from 1 to 2000, preferably from 1 to 10;
   n is a number from 0 to 1999, preferably from 49 to 149, and
   R' is a monovalent radical of formula -C_{q}H_{2q}L in which q is a number from 2 to 8 and L is an aminofuctional group selected from the following:

      -NR"-CH₂-CH₂-N(R")₂

      -N(R")₂

      -N⁺(R")₃A⁻

      -N⁺H(R")₂A⁻

      -N⁺H(R")₂A⁻

      -N⁺H₂(R")A⁻

      -N(R")-CH₂-CH₂-N⁺H₂(R")A⁻
   in which R is selected from H, phenyl, benzyl, or a saturated monovalent hydrocarbon radical, e.g. C₁₋₂₀ alkyl, and A is a halide ion, e.g. chloride or bromide.
   Suitable amino functional silicones corresponding to the above formula include those polysiloxanes termed "trimethylsilylamodimethicone" as depicted below, and which are sufficiently water insoluble so as to be useful in compositions of the invention:

   Si(CH₃)₃-O-[Si(CH₃)₂-O-]ₓ-[Si (CH₃) (R-NH-CH₂CH₂NH₂)-O-]_{y}-Si(CH₃)₃

   wherein x + y is a number from about 50 to about 500, and wherein R is an alkylene group having from 2 to 5 carbon atoms. Preferably, the number x + y is in the range of from about 100 to about 300.
(iii) quaternary silicone polymers having the general formula:

   {(R¹)(R²)(R³)N⁺CH₂CH(OH)CH₂O(CH₂)₃[Si(R⁴)(R⁵)-O-]ₙ-Si(R⁶)(R⁷)-(CH₂)₃-O-CH₂CH(OH)CH₂N⁺(R⁸)(R⁹)(R¹⁰)} (X⁻)₂

   wherein R¹ and R¹⁰ may be the same or different and may be independently selected from H, saturated or unsaturated long or short chain alk(en)yl, branched chain alk(en)yl and C₅-C₈ cyclic ring systems;
   R² thru' R⁹ may be the same or different and may be independently selected from H, straight or branched chain lower alk(en)yl, and C₅-C₈ cyclic ring systems;
   n is a number within the range of about 60 to about 120, preferably about 80, and
   X⁻ is preferably acetate, but may instead be for example halide, organic carboxylate, organic sulphonate or the like. Suitable quaternary silicone polymers of this class are described in EP-A-0 530 974.

Amino functional silicones suitable for use in shampoos and conditioners of the invention will typically have a mole % amine functionality in the range of from about 0.1 to about 8.0 mole %, preferably from about 0.1 to about 5.0 mole %, most preferably from about 0.1 to about 2.0 mole %. In general the amine concentration should not exceed about 8.0 mole % since we have found that too high an amine concentration can be detrimental to total silicone deposition and therefore conditioning performance.

The viscosity of the amino functional silicone is not particularly critical and can suitably range from about 100 to about 500,000 cst.

Specific examples of amino functional silicones suitable for use in the invention are the aminosilicone oils DC2-8220, DC2-8166, DC2-8466, and DC2-8950-114 (all ex Dow Corning), and GE 1149-75, (ex General Electric Silicones).

Also suitable are emulsions of amino functional silicone oils with non ionic and/or cationic surfactant.

Suitably such pre-formed emulsions will have an average amino functional silicone droplet size in the shampoo composition of less than 30, preferably less than 20, more preferably less than 10 µm. Again, we have found that reducing the droplet size generally improves conditioning performance. Most preferably the average amino functional silicone droplet size in the composition is less than 2 µm ideally it ranges from 0.01 to 1 µm.

Pre-formed emulsions of amino functional silicone are also available from suppliers of silicone oils such as Dow Corning and General Electric. Specific examples include DC929 Cationic Emulsion, DC939 Cationic Emulsion, and the non-ionic emulsions DC2-7224, DC2-8467, DC2-8177 and DC2-8154 (all ex Dow Corning).

An example of a quaternary silicone polymer useful in the present invention is the material K3474, ex Goldschmidt.

For shampoo compositions according to the invention intended for the treatment of "mixed" hair (i.e. greasy roots and dry ends), it is particularly preferred to use a combination of amino functional and non-amino functional silicone in compositions of the invention, especially when these are in the form of shampoo compositions. In such a case, the weight ratio of amino functional silicone to non-amino functional silicone will typically range from 1:2 to 1:20, preferably 1:3 to 1:20, more preferably 1:3 to 1:8. The total amount of silicone incorporated into compositions of the invention depends on the level of conditioning desired and the material used. A preferred amount is from 0.01 to 10 wt% although these limits are not absolute. The lower limit is determined by the minimum level to achieve conditioning and the upper limit by the maximum level to avoid making the hair and/or skin unacceptably greasy.

We have found that a total amount of silicone of from 0.3 to 5, preferably 0.5 to 3 wt% is a suitable level.

The viscosity of silicones and silicone emulsions can be measured by means of a glass capillary viscometer as set out further in Dow Corning Corporate Test Method CTM004, July 20 1970.

In compositions comprising silicone, it is preferred that a suspending agent for the silicone also be present. Suitable suspending agents are as described hereinabove.

In a preferred embodiment of the present invention, the shampoo compositions are substantially free of silicone.

The invention will now be further illustrated by the following, non-limiting Examples.

### EXAMPLES

### Example 1

The following shampoo formulation was prepared:

| | % wt |
|---|---|
| | (active ingredient) |
| Carbopol 980 | 0.4 |
| SLES 2EO | 14.0 |
| CAPB | 2.0 |
| Jaguar C13S | 0.1 |
| Perfume | 0.6 |
| Glydant plus* | 0.2 |
| Soybean oil | 3.0 |
| Sodium chloride | 1.0 |
| BHT | 0.24 |
| Minors and water | to 100% |

| | |
|---|---|
| * Glydant plus is a commercially available preservative material. | |

Processing was carried out at ambient temperature. The pH was adjusted to 6.5 with sodium hydroxide solution. Six samples were prepared, each containing a different oil droplet size.

The samples containing the three smaller droplet sizes were prepared by preemulsifying the soybean oil using a Rannie pressure homogeniser operating at different pressures, namely 50, 175 and 700 bar. This resulted in droplet sizes of 0.79, 0.45 and 0.30 µm respectively. The BHT was dissolved in the oil beforehand.

The samples containing the three larger droplet sizes were prepared as follows:
Ystral: By adding the oil direct to the shampoo and emulsifying *in. situ.* using a Ystral high shear mixer. In this case the order of addition was as shown above, with the emulsification taking place before addition of salt. This resulted in a D_{3,2} mean oil droplet size of 1.51 µm.
Medium speed: As above but using a Heidolph stirrer at a tip speed of 2 m/s instead of Ystral. This resulted in a D_{3,2} mean oil droplet size of 3.05 µm.
Gentle: As above but stirring in the oil with a spatula, then "rolling" for a total of 30 minutes. This resulted in a D_{3,2} mean oil droplet size of 10.67 µm.

The six samples containing oil droplets of differing sizes were tested for stability and efficiency of deposition. Deposition was measured by treating hair switches (of proximal hair) with the shampoos and then, after drying the switches, extracting the oil from the hair with a solvent and analysing by GC.

The stability of the samples was assessed by monitoring changes in viscosity over time. The viscosity was measured at ambient immediately after manufacture using a Brookfield spindle 4 at 20 RPM. Samples of the products were also put on store at 45°C for 2 weeks. After this period they were allowed to cool to ambient and the viscosity measured again.

The relationship between deposition and droplet size (in µm) and stability in term of viscosity and droplet size is given in the following table.

| Size (D_{3,2}) | Deposition efficiency (%) | Initial viscosity | Final viscosity |
|---|---|---|---|
| 10.67 | 8.29 | 6,400 | 6,600 |
| 3.05 | 8.75 | 6,400 | 6,400 |
| 1.51 | 10.71 | 5,100 | 4,700 |
| 0.79 | 33.7 | 5,500 | 4,400 |
| 0.45 | 42.1 | 5,300 | 3,500 |
| 0.30 | 64.6 | 5,300 | 1,700 |

The storage conditions under which the samples were kept were stringent, i.e. 45°C. Even under these conditions, the compositions with a droplet size of greater that 0.4 µm were relatively stable over a two week period.

## Claims

1. An aqueous shampoo composition comprising, in addition to water:
i) a cleansing surfactant;
ii) A dispersed non-volatile, water-insoluble, oily conditioning agent comprising a triglyceride, the average D_{3,2} droplet size of which is in the range from 0.8 co 4 µm; and
iii) a cationic polymer.

2. A composition as claimed in claim 1, in which the D_{3,2} average droplet size of the oily conditioning component is in the range from 1.0 to 3.5 µm.

3. A composition according to claim 1 or claim 2, in which the oily conditioning agent is present in an amount of from 0.05 to 10 wt%.

4. A composition according to any preceding claim, in which the cationic polymer is present in an amount of from 0.01 to 5 wt%.

5. A composition according to any preceding claim, in which the cationic polymer is a polysaccharide polymer having a cationic charge density of from 0.1 to 4 meq/g.

6. A composition according to claim 5, in which the cationic polymer is selected from cationic cellulose and cationic guar derivatives.

7. A composition according to any preceding claim, further comprising from 0.1 to 5 wt% of a polymeric suspending agent for the oily conditioning agent.

8. A composition according to claim 7, in which the suspending agent is selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters.

9. A composition according to any preceding claim, in which the cleansing surfactant comprises from 5 to 30 wt% of one or more anionic cleansing surfactants selected from alkyl sulphates, alkyl ether sulphates, alkaryl sulphonates, alkanoyl isethionates, alkyl succinates, alkyl sulphosuccinates, N-alkyl sarcosinates, alkyl phosphates, alkyl ether phosphates, alkyl ether carboxylates, and alpha-olefin sulphonates, especially their sodium, magnesium, ammonium and mono-, di- and triethanolamine salts, and mixture thereof.

## Patentansprüche

1. Wässeriges Haarwaschmittel, das zusätzlich zu Wasser umfasst:
i) ein reinigendes Tensid
ii) ein dispergiertes, nichtflüchtiges, wasserunlösliches, öliges Pflegemittel mit einem Triglycerid, dessen durchschnittliche D_{3,2} Tröpfchengröße im Bereich von 0,8 bis 4 µm liegt, und
iii) ein kationisches Polymer.

2. Zusammensetzung nach Anspruch 1, in der die durchschnittliche D_{3.2} Tröpfchengröße der öligen Pflegekomponente im Bereich von 1,0 bis 3,5 µm liegt.

3. Zusammensetzung nach Anspruch 1 oder Anspruch 2, in der das ölige Pflegemittel in einer Menge von 0,05 bis 10 Gew.-% vorliegt.

4. Zusammensetzung nach einem der vorstehenden Ansprüche, in der das kationische Polymer in einer Menge von 0,01 bis 5 Gew.% vorliegt.

5. Zusammensetzung nach einem der vorstehenden Ansprüche, in der das kationische Polymer ein Polysaccharidpolymer mit einer kationischen Ladungsdichte von 0,1 bis 4 mÄq/g ist.

6. Zusammensetzung nach Anspruch 5, in der das kationische Polymer aus kationischer Cellulose und kationischen Guarderivaten ausgewählt ist.

7. Zusammensetzung nach einem der vorstehenden Ansprüche, die außerdem 0,1 bis 5 Gew.-% eines polymeren Suspendiermittels für das ölige Pflegemittel umfasst.

8. Zusammensetzung nach Anspruch 7, in der das Suspendiermittel aus Polyacrylsäuren, vernetzten Polymeren von Acrylsäure, Copolymeren von Acrylsäure mit einem hydrophoben Monomer, Copolymeren von carbonsäurehaltigen Monomeren und Acrylestern, vernetzten Copolymeren von Acrylsäure und Acrylatestern ausgewählt ist.

9. Zusammensetzung nach einem der vorstehenden Ansprüche, in der das reinigende Tensid 5 bis 30 Gew.-% eines oder mehrerer anionischer reinigender Tenside umfasst, ausgewählt aus Alkylsulfaten, Alkylethersulfaten, Alkarylsulfonaten, Alkanoylisethionaten, Alkylsuccinaten, Alkylsulfosuccinaten, N-Alkylsarcosinaten, Alkylphosphaten, Alkyletherphosphaten, Alkylethercarboxylaten und α-Olefinsulfonaten, insbesondere ihren Natrium-, Magnesium-, Ammonium und Mono-, Di- und Triethanolaminsalzen, und Mischungen davon.

## Revendications

1. Composition de shampooing aqueuse comprenant, outre l'eau :
i) un tensioactif de lavage ;
ii) un agent conditionneur huileux, insoluble dans l'eau, non volatil, dispersé, comprenant un triglycéride, dont la dimension des gouttelettes moyenne D_{3,2} est dans la plage de 0,8 à 4 *µ*m ; et
iii) un polymère cationique.

2. Composition selon la revendication 1, dans laquelle la dimension des gouttelettes moyenne D_{3,2} du composant conditionneur huileux est dans la plage de 1,0 à 3,5 µm.

3. Composition selon la revendication 1 ou la revendication 2, dans laquelle l'agent conditionneur huileux est présent dans une quantité de 0,05 à 10 % en poids.

4. Composition selon l'une quelconque des revendications précédentes, dans laquelle le polymère cationique est présent dans une quantité de 0,01 à 5 % en poids.

5. Composition selon l'une quelconque des revendications précédentes, dans laquelle le polymère cationique est un polymère polysaccharidique ayant une densité de charge cationique de 0,1 à 4 meq/g.

6. Composition selon la revendication 5, dans laquelle le polymère cationique est choisi parmi la cellulose cationique et les dérivés de guar cationiques.

7. Composition selon l'une quelconque des revendications précédentes, comprenant en outre de 0,1 à 5 % en poids d'un agent de suspension polymérique pour l'agent conditionneur huileux.

8. Composition selon la revendication 7, dans laquelle l'agent de suspension est choisi parmi les acides polyacryliques, les polymères réticulés d'acide acrylique, les copolymères d'acide acrylique avec un monomère hydrophobe, les copolymères de monomères contenant un acide carboxylique et d'esters acryliques, les copolymères réticulés d'acide acrylique et d'esters d'acrylate.

9. Composition selon l'une quelconque des revendications précédentes, dans laquelle le tensioactif de lavage comprend de 5 à 30 % en poids d'un ou plusieurs tensioactifs de lavage anioniques choisis parmi les alkylsulfates, les alkyléthersulfates, les alkarylsulfonates, les alcanoyliséthionates, les alkylsuccinates, les alkylsulfosuccinates, les N-alkylsarcosinates, les alkylphosphates, les alkylétherphosphates, les alkyléthercarboxylates, et les sulfonates d'alpha-oléfine, en particulier leurs sels de sodium, de magnésium, d'ammonium et de monoéthanolamine, de diéthanolamine et de triéthanolamine, et des mélanges de ceux-ci.
